# EUROPEAN PATENT APPLICATION

(11) **EP 0 970 697 A1**
(43) Date of publication of application: **12.01.2000**
(21) Application number: 98943010.3
(22) Date of filing: 16.09.1998
(51) Int. Cl.: A61K 31/557, C07D 307/93

(54) **CERVICAL MATURING AGENT**

(30) Priority: 17.09.1997 JP 25250597
(71) Applicant: TORAY INDUSTRIES, INC., Tokyo 103-8666 (JP)
(72) Inventor: OCHI, Yasuo, Kamakura-shi Kanagawa 248-0036 (JP); YAMADA, Naohiro, Kamakura-shi, Kanagawa 248-0034 (JP); WAKITA, Hisanori, Kanagawa 253-0085 (JP); MORIYAMA, Masami, Yokohama-shi, Kanagawa 232-0064 (JP)
(74) Representative: Coleiro, Raymond
(86) International application number: JP9804165
(87) International publication number: WO9913881

(57) **Abstract**

A cervical maturing agent containing a prostaglandin I derivative as the active ingredient. It exerts excellent maturing effect on the cervical canal without causing any uterine contraction, which makes it useful as a cervical maturing agent.

## Description

### Technical Field

The present invention relates to a cervical ripening agent, and new application of prostaglandin I derivatives.

### Background Art

Parturition requires both softening of the cervical canal to sufficiently expand it for extracting a fetus, and contraction of the myometrium which is the uterine smooth muscle. The cervical canal is securely closed for continuation of pregnancy up to the latter stage thereof to prevent abortion and premature delivery. On the other hand, for extraction of a fetus, softening of the cervical canal proceeds after the 36th week of pregnancy accompanied with shortening (effacement) of the cervical canal. This is a change referred to as so-called "cervical ripening". The cervical ripening is an important factor which determines the process of extracting a fetus, and smooth proceeding of the ripening is required for normal proceeding of delivery. Therefore, as a pre-stage for parturition before the start of contraction of the myometrium, sufficient ripening of the cervical canal is required for safe extraction.

At present, the number of women who give birth to children in their 20s decreases, and the number of women who give birth to children in their 30s or 40s increases. It is estimated that ten years after, the number of women who give birth to children in their 20s will further decrease, and the number of women who give birth to children in their 30s will further increase. The greatest problem in the field of obstetrics and gynecology is rigidity of the parturient canal, in its turn insufficient cervical ripening, which occurs with advances in the ages of women which give birth to children. With a low degree of cervical ripening, the cervical opening is dilated by a mechanical method, or the cervical canal is softened by administration of a cervical ripening agent.

As a medicine for accelerating cervical ripening, prostaglandin E₂ (abbreviated to "PGE₂" hereinafter), and dehydroepiandrosterone sulfate (abbreviated to "DHAS" hereinafter) are clinically widely used at present. However, PGE₂ has the problem of possibly causing excessive contraction of the myometrium. DHAS has no adverse effect of causing excessive contraction of the myometrium, but has the problem of requiring intravenous administration for ripening the cervical canal, and requiring administration for a relatively long period. On the other hand, it has been reported that beraprost sodium (abbreviated to "BPS" hereinafter) which is a prostaglandin I₂ derivative having no oxytocic action exhibits a cervical ripening action in mice. However, a high dosage of 100 µg to 200 µg (about 2 to 4 mg per kg of weight) is required, which exceeds a general clinical dosage largely, thereby causing the problem of the possibility that an adverse effect is manifested.

An object of the present invention is to provide an excellent cervical ripening accelerator for sufficiently ripening the cervical canal without causing uterine contraction.

### Disclosure of Invention

The present invention relates to a cervical ripening agent containing as an active ingredient a prostaglandin I derivative.

### Brief Description of the Drawings

Fig. 1 shows changes in maximum tension in extension of the cervical canal, which were caused by intravaginal administration of test medicines to guinea pigs.
Fig. 2 shows changes in the slope in extension of the cervical canal, which were caused by intravaginal administration of test medicines to guinea pigs.

### Best Mode for Carrying Out the Invention

Although prostaglandin I derivatives of the present invention include prostaglandin I₂ derivatives, particularly metaphenylene, carbacyclin and isocarbacyclin type prostaglandin I₂ derivatives, the derivatives are not limited to these derivatives. Metaphenylene prostaglandin I₂ derivatives are preferably used, and 4,8-inter-m-phenylene prostaglandin I₂ derivatives represented by the following formula (I) or pharmacologically acceptable salts thereof are more preferably used. 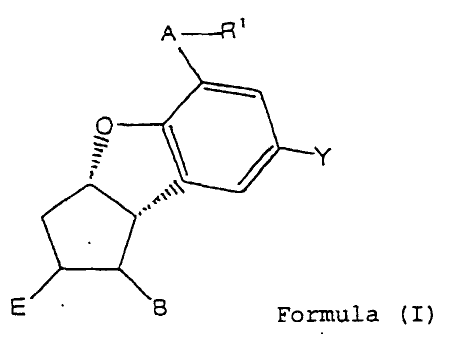 [wherein R¹ represents the following:
(A) COOR² wherein R² is:
   1) hydrogen or a pharmacologically acceptable cation;
   2) normal alkyl having 1 to 12 carbon atoms, or branched alkyl having 3 to 14 carbon atoms;
   3) -Z-R³
      wherein Z is a valence bond or normal or branched alkylene represented by CₜH₂ₜ wherein t represents an integer of 1 to 6, and R³ represents cycloalkyl having 3 to 12 carbon atoms or substituted cycloalkyl having 3 to 12 carbon atoms and 1 to 3 substituents R⁴ which is hydrogen or alkyl having 1 to 5 carbon atoms;
   4) -(CH₂CH₂O)ₙCH₃
      wherein n is an integer of 1 to 5;
   5) -Z-Ar¹
      wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein a substituent is at least one of chlorine, bromine, fluorine, iodine, trifluoromethyl, alkyl having 1 to 4 carbon atoms, nitro, cyano, methoxy, phenyl, phenoxy, p-acetoamidobenzamide, -CH=N-NH-C(=0)-NH₂, -NH-C(=O)-Ph, -NH-C(=0)-CH₃ and -NH-C(=O)-NH₂);
   6) -CₜH₂ₜCOOR⁴
      wherein CₜH₂ₜ and R⁴ are defined as the same as the above;
   7) -CₜH₂ₜN(R⁴)₂
      wherein CₜH₂ₜ and R⁴ are defined as the same as the above;
   8) -CH(R⁵)-C(=O)-R⁶
      wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
   9) -CₚH₂ₚ-W-R⁷
      wherein W is -CH=CH-, -CH=CR⁷- or -C≡C-, and R⁷ is hydrogen or normal or branched alkyl or aralkyl having 1 to 30 carbon atoms, and p is an integer of 1 to 5; or
   10) -CH(CH₂OR⁸)₂
      wherein R⁸ is alkyl or acyl having 1 to 30 carbon atoms;
(B) -CH₂OH;
(C) -C(=O)N(R⁹)₂
   wherein R⁹ is hydrogen, normal alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as in (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substitutent is defined as the sane as in (A) 5)), or aralkyl having 7 to 12 carbon atoms, two R⁹ groups may be the same or different, and when one of the R⁹ groups is -SO₂R¹⁰, the other R⁹ is not -SO₂R¹⁰; or
(D) -CH₂OTHP (THP is a tetrahydropyranyl group);
   A is the following:
      1) -(CH₂)ₘ-;
      2) -CH=CH-CH₂-;
      3) -CH₂-CH=CH-;
      4) -CH₂-O-CH₂-;
      5) -CH=CH-;
      6) -O-CH₂-; or
      7) -C≡C-;
         wherein m represents an integer of 1 to 3;
   Y is hydrogen, alkyl having 1 to 4 carbon atoms, chlorine, bromine, fluorine, formyl, methoxy or nitro;
   B is -X-C(R¹¹)(R¹²)OR¹³
      wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxyethyl, or t-butyl; X is the following:
      1) -CH₂-CH₂-;
      2) -CH=CH-; or
      3) -C≡C-; and
   R¹² is the following:
      1) normal alkyl having 1 to 12 carbon atoms, or branched alkyl having 3 to 14 carbon atoms;
      2) -Z-Ar²
         wherein Z is defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
      3) -CₜH₂ₜOR¹⁴
         wherein CₜH₂ₜ is defined as the same as the above, and R¹⁴ represents normal alkyl having 1 to 6 carbon atoms, branched alkyl having 3 to 6 carbon atoms, phenyl, phenyl substituted by at least one chlorine, bromine, fluorine, iodine, trifluoromethyl, alkyl having 1 to 4 carbon atoms, nitro, cyano, methoxy, phenyl or phenoxy, cyclopentyl, cyclohexyl, or cyclopentyl or cyclohexyl substituted by 1 to 4 normal alkyl groups having 1 to 4 carbon atoms;
      4) -Z-R³
         wherein Z and R³ are defined as the same as the above;
      5) -CₜH₂ₜ-CH=C(R¹⁵)R¹⁶
         wherein CₜH₂ₜ is defined as the same as the above, and R¹⁵ and R¹⁶ each represent hydrogen, methyl, ethyl, propyl, or butyl; or
      6) -CᵤH₂ᵤ-C=C-R¹⁷
         wherein u is an integer of 1 to 7, CᵤH₂ᵤ represents normal or branched alkyl, and R¹⁷ represents normal alkyl having 1 to 6 carbon atoms (when m = 3, branched ethylene C₂H₄ and methyl as R¹⁷ are excluded) ;
   E is hydrogen or -OR¹⁸
      wherein R¹⁸ represents acyl having 1 to 12 carbon atoms, aroyl having 7 to 15 carbon atoms, or R² (wherein R² is defined as the same as the above); and
   the formula represents the d, l or dl form].

Examples of pharmacologically acceptable salts include alkali metal salts such as sodium salts, potassium salts, and the like; alkali earth metal salts such as calcium salts, magnesium salts, and the like; amine salts such as methylamine salts, dimethylamine salts, trimethylamine salts, methylpiperidine salts, ethanolamine salts, diethanolamine salts, triethanolamine salts, lysine salts, and the like; ammonium salts; basic amine acid salts; and the like.

Although at least one prostaglandin I derivative or a salt thereof can be administered to the uterus as the cervical ripening agent of the present invention, an excipient, a stabilizer, etc., which are generally used in preparation of medicines, can also be added to the cervical ripening agent. Examples of such additives include animal oil, plant oil, paraffin, gum arabic, starch, saccharides such as lactose, sucrose, glucose, dextrin, mannitol, and the like; inorganic acid salts such as calcium carbonate, calcium sulfate, and the like; organic acid salts such as sodium citrate, sodium lactate, magnesium stearate, and the like; water-soluble polymers such as methyl cellulose, gelatin, polyethylene glycol, polyvinyl alcohol, polyvinylpyrrolidone, hydroxyethyl cellulose, hydroxypropyl cellulose, and the like; alcohols such as ethanol, glycerin, propylene glycol, sorbitol, and the like; surfactants such as sorbitan fatty acid esters, polyoxyethylene sorbitan fatty acid esters, polyoxyethylene fatty acid esters, glycerin fatty acid esters, and the like.

The cervical ripening agent of the present invention can be used in various forms. Examples of the forms include conventional forms such as liquids such as a vaginal epipastic, semi-solids such as an ointment, a cream, a gel, and the like; solids such as a vaginal tablet, a vaginal capsule, a pessary, a vaginal suppository, and the like. The cervical ripening agent can also be used as an injection for hypodermic injection, intravenous injection, local injection or the like. Since the prostaglandin I₂ derivatives of the present invention are compounds which can be orally administered, forms such as a tablet, a powder, granules, a pill, a capsule, and the like can also be used.

The cervical ripening agent of the present invention can be administered in one or divided doses of 0.0001 to 1000 mg/adult, preferably 0.001 to 500 mg/adult, depending upon the symptoms, the age, uterine conditions, the form of the agent administered, etc.

The cervical ripening agent of the present invention can also be used for animals other than humans. Namely, by administering the agent to an animal other than the human, it is possible to ripen the cervical canal, and accelerate delivery of a fetus. The cervical ripening agent can thus be applied to curing and treatment in pregnancy.

### [Examples]

In order to describe the present invention in further detail, examples are described below.

### Example 1

### Cervical ripening action:

The cervical ripening actions of compounds of formula (I) were examined by using Hartley mature guinea pigs (nine-week-old, clean animals). As test medicines, the compounds shown in Table 1 were used.

In order to compare with these compounds, the action of BPS was also tested. Experiment was carried out for 4 to 14 samples per group.

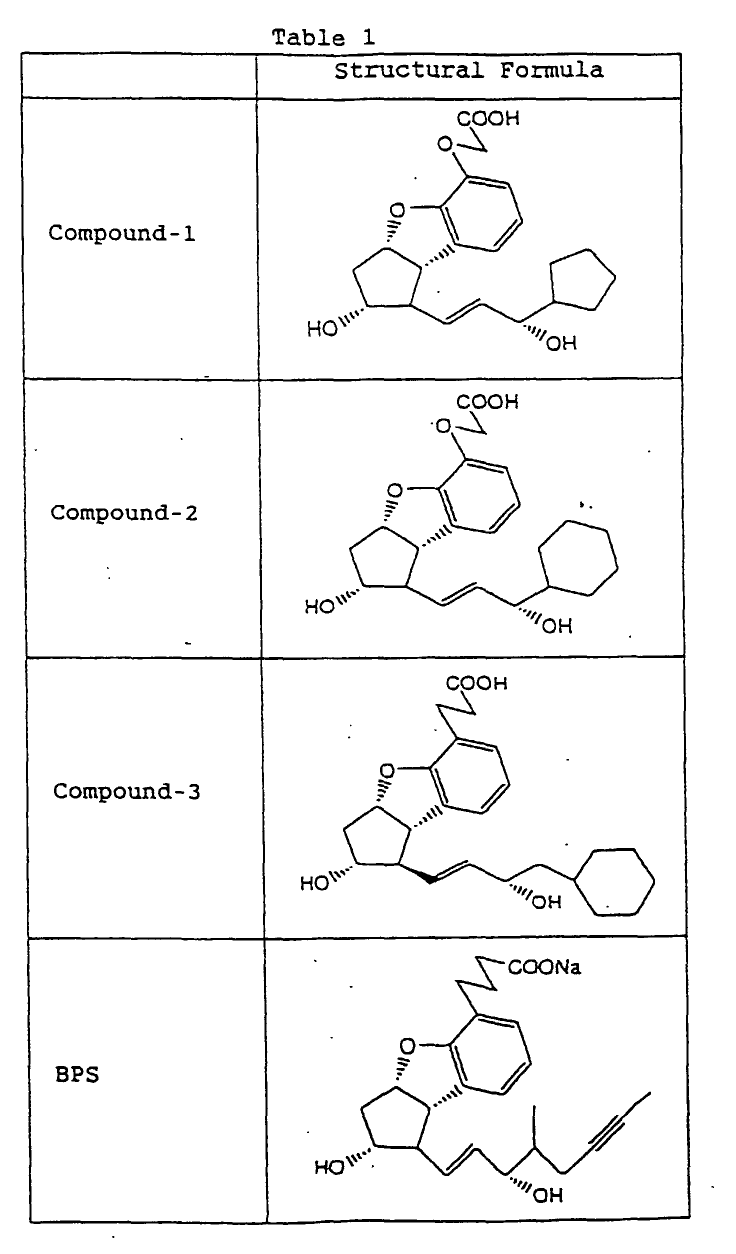

In the test, 100 µl of a test medicine or a base thereof was administered to the vagina at 9 o'clock and 17 o'clock, and 9 o'clock on the next day, and a guinea pig was dissected to extract the cervical canal 4 hours after the final administration. Then, the cervical canal was extended at a rate of 2 mm/min to measure tension generated in the tissue with time. As an index for cervical ripening, the maximum tension generated up to breakage of the tissue, and the slope obtained by dividing the maximum tension by the distance of extension were examined. It is thought that decreases in these values indicate ripening of the cervical canal. A test medicine was dissolved in phosphate buffered saline, and then mixed with 3% hydroxypropyl cellulose.

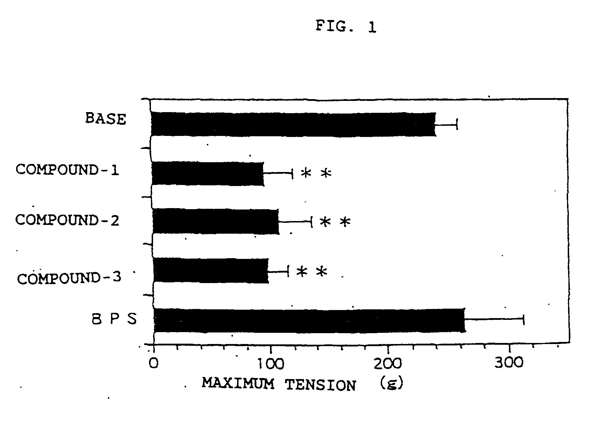

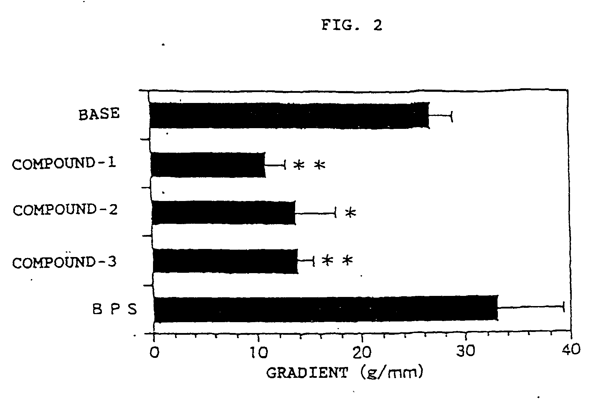

Figs. 1 and 2 show changes in the maximum tension and the slope, respectively, caused by the test medicines. (*: P < 0.05, **: P < 0.01, Dunnett's method, comparison with the base administration group). BPS (10µg) showed no cervical ripening action. On the other hand, compounds 1, 2 and 3 (10µg) showed the significant cervical ripening action, as compared with the base administration group.

These results reveal that compounds 1, 2 and 3 have the excellent cervical ripening action.

### Industrial Applicability

The cervical ripening agent of the present invention has an excellent softening action on the cervical canal without causing uterine contraction, and is very useful as a cervical ripening agent.

## Claims

1. A cervical ripening agent comprising a prostaglandin I derivative as an active ingredient.

2. A cervical ripening agent according to Claim 1, wherein the prostaglandin I derivative is a prostaglandin I₂ derivative.

3. A cervical ripening agent according to Claim 2, wherein the prostaglandin I₂ derivative is a metaphenylene, carbacyclin or isocarbacyclin type prostaglandin I₂ derivative.

4. A cervical ripening agent according to Claim 3, comprising as an active ingredient 4,8-inter-m-phenylene prostaglandin I₂ derivative as the metaphenylene type prostaglandin I₂ derivative or a pharmacologically acceptable salt thereof, which is represented by the following formula(I): 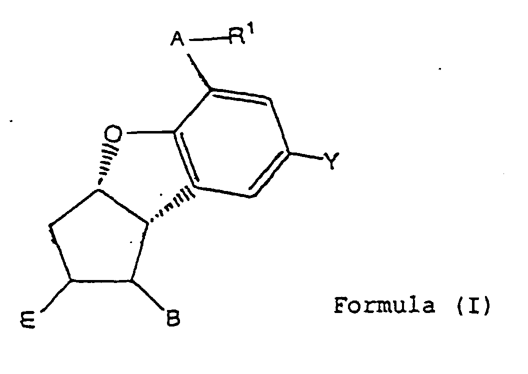 (wherein R¹ represents the following:
(A) COOR² wherein R² is:
1) hydrogen or a pharmacologically acceptable cation;
2) normal alkyl having 1 to 12 carbon atoms, or branched alkyl having 3 to 14 carbon atoms;
3) -Z-R³
wherein Z is a valence bond or normal or branched alkylene represented by CₜH₂ₜ wherein t represents an integer of 1 to 6, and R³ represents cycloalkyl having 3 to 12 carbon atoms or substituted cycloalkyl having 3 to 12 carbon atoms and 1 to 3 substituents R⁴ which is hydrogen or alkyl having 1 to 5 carbon atoms;
4) -(CH₂CH₂O)ₙCH₃
wherein n is an integer of 1 to 5;
5) -Z-Ar¹
wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein a substituent is at least one of chlorine, bromine, fluorine, iodine, trifluoromethyl, alkyl having 1 to 4 carbon atoms, nitro, cyano, methoxy, phenyl, phenoxy, p-acetoamidobenzamide, -CH=N-NH-C(=O)-NH₂, -NH-C(=O)-Ph, -NH-C(=O)-CH₃ and -NH-C(=O)-NH₂);
6) -CₜH₂ₜCOOR⁴
wherein CₜH₂ₜ and R⁴ are defined as the same as the above;
7) -CₜH₂ₜN(R⁴)₂
wherein CₜH₂ₜ and R⁴ are defined as the same as the above;
8) -CH(R⁵)-C(=O)-R⁶
wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
9) -CₚH₂ₚ-W-R⁷
wherein W is -CH=CH-, -CH=CR⁷- or -C≡C-, and R⁷ is hydrogen or normal or branched alkyl or aralkyl having 1 to 30 carbon atoms, and p is an integer of 1 to 5; or
10) -CH(CH₂OR⁸)₂
wherein R⁸ is alkyl or acyl having 1 to 30 carbon atoms;
(B) -CH₂OH;
(C) -C(=O)N(R⁹)₂
wherein R⁹ is hydrogen, normal alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as in (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substitutent is defined as the same as in (A) 5)), or aralkyl having 7 to 12 carbon atoms, two R⁹ groups may be the same or different, and when one of the R⁹ groups is -SO₂R¹⁰, the other R⁹ is not -SO₂R¹⁰; or
(D) -CH₂OTHP (THP is a tetrahydropyranyl group);
A is the following:
1) -(CH₂)ₘ-;
2) -CH=CH-CH₂-;
3) -CH₂-CH=CH-;
4) -CH₂-O-CH₂-;
5) -CH=CH-;
6) -O-CH₂-; or
7) -C≡C-;
wherein m represents an integer of 1 to 3;
Y is hydrogen, alkyl having 1 to 4 carbon atoms, chlorine, bromine, fluorine, formyl, methoxy or nitro;
B is -X-C(R¹¹)(R¹²)OR¹³
wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxyethyl, or t-butyl; X is the following:
1) -CH₂-CH₂-;
2) -CH=CH-; or
3) -C≡C-; and
R¹² is the following:
1) normal alkyl having 1 to 12 carbon atoms, or branched alkyl having 3 to 14 carbon atoms;
2) -Z-Ar²
wherein Z is defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
3) -CₜH₂ₜOR¹⁴
wherein CₜH₂ₜ is defined as the same as the above, and R¹⁴ represents normal alkyl having 1 to 6 carbon atoms, branched alkyl having 3 to 6 carbon atoms, phenyl, phenyl substituted by at least one chlorine, bromine, fluorine, iodine, trifluoromethyl, alkyl having 1 to 4 carbon atoms, nitro, cyano, methoxy, phenyl or phenoxy, cyclopentyl, cyclohexyl, or cyclopentyl or cyclohexyl substituted by 1 to 4 normal alkyl groups having 1 to 4 carbon atoms;
4) -Z-R³
wherein Z and R³ are defined as the same as the above;
5) -CₜH₂ₜ-CH=C(R¹⁵)R¹⁶
wherein CₜH₂ₜ is defined as the same as the above, and R¹⁵ and R¹⁶ each represent hydrogen, methyl, ethyl, propyl, or butyl; or
6) -CᵤH₂ᵤ-C≡C-R¹⁷
wherein u is an integer of 1 to 7, CᵤH₂ᵤ represents normal or branched alkyl, and R¹⁷ represents normal alkyl having 1 to 6 carbon atoms (when m = 3, branched ethylene C₂H₄ and methyl as R¹⁷ are excluded);
E is hydrogen or -OR¹⁸
wherein R¹⁸ represents acyl having 1 to 12 carbon atoms, aroyl having 7 to 15 carbon atoms, or R² (wherein R² is defined as the same as the above); and
the formula represents the d, l or dl form].

5. A cervical ripening agent according to Claim 4, wherein A is - (CH₂)ₘ-, -CH=CH-CH₂-, -CH₂-CH=CH-, -CH₂-O-CH₂-, -CH=CH-, -O-CH₂-, or -C≡C- (m is 1 or 2).

6. A cervical ripening agent according to Claim 4, wherein A is -(CH₂)₂-, -CH=CH-, or -O-CH₂-.

7. A cervical ripening method comprising administering an effective amount of prostaglandin I derivative.

8. A cervical ripening method according to Claim 7, wherein the prostaglandin I derivative is a prostaglandin I₂ derivative.

9. A cervical ripening method according to Claim 8, wherein the prostaglandin I₂ derivative is a metaphenylene, carbacyclin or isocarbacyclin type prostaglandin I₂ derivative.

10. A cervical ripening method according to Claim 9, comprising as an active ingredient 4,8-inter-m-phenylene prostaglandin I₂ derivative as the metaphenylene type prostaglandin I₂ derivative or a pharmacologically acceptable salt thereof, which is represented by the following formula(I): 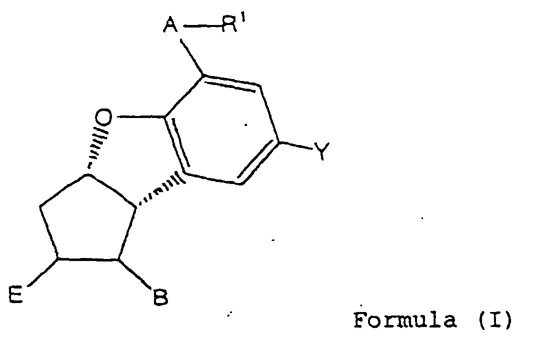 [wherein R¹ represents the following:
(A) COOR² wherein R² is:
1) hydrogen or a pharmacologically acceptable cation;
2) normal alkyl having 1 to 12 carbon atoms, or branched alkyl having 3 to 14 carbon atoms;
3) -Z-R³
wherein Z is a valence bond or normal or branched alkylene represented by CₜH₂ₜ wherein t represents an integer of 1 to 6, and R³ represents cydloalkyl having 3 to 12 carbon atoms or substituted cycloalkyl having 3 to 12 carbon atoms and 1 to 3 substituents R⁴ which is hydrogen or alkyl having 1 to 5 carbon atoms;
4) -(CH₂CH₂O)ₙCH₃
wherein n is an integer of 1 to 5;
5) -Z-Ar¹
wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein a substituent is at least one of chlorine, bromine, fluorine, iodine, trifluoromethyl, alkyl having 1 to 4 carbon atoms, nitro, cyano, methoxy, phenyl, phenoxy, p-acetoamidobenzamide, -CH=N-NH-C(=O)-NH₂, -NH-C(=O)-Ph, -NH-C(=O)-CH₃ and -NH-C(=O)-NH₂);
6) -CₜH₂ₜCOOR⁴
wherein CₜH₂ₜ and R⁴ are defined as the same as the above;
7) -CₜH₂ₜN(R⁴)₂
wherein CₜH₂ₜ and R⁴ are defined as the same as the above;
8) -CH(R⁵)-C(=O)-R⁶
wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
9) -CₚH₂ₚ-W-R⁷
wherein W is -CH=CH-, -CH=CR⁷- or -C≡C-, and R⁷ is hydrogen or normal or branched alkyl or aralkyl having 1 to 30 carbon atoms, and p is an integer of 1 to 5; or
10) -CH(CH₂OR⁸)₂
wherein R⁸ is alkyl or acyl having 1 to 30 carbon atoms;
(B) -CH₂OH;
(C) -C(=O)N(R⁹)₂
wherein R⁹ is hydrogen, normal alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as in (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substitutent is defined as the same as in (A) 5)), or aralkyl having 7 to 12 carbon atoms, two R⁹ groups may be the same or different, and when one of the R⁹ groups is -SO₂R¹⁰, the other R⁹ is not -SO₂R¹⁰; or
(D) -CH₂OTHP (THP is a tetrahydropyranyl group);
A is the following:
1) -(CH₂)ₘ-;
2) -CH=CH-CH₂-;
3) -CH₂-CH=CH-;
4) -CH₂-O-CH₂-;
5) -CH=CH-;
6) -O-CH₂-; or
7) -C≡C-;
wherein m represents an integer of 1 to 3;
Y is hydrogen, alkyl having 1 to 4-carbon atoms, chlorine, bromine, fluorine, formyl, methoxy or nitro;
B is -X-C(R¹¹)(R¹²)OR¹³
wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxyethyl, or t-butyl; X is the following:
1) -CH₂-CH₂-;
2) -CH=CH-; or
3) -C≡C-; and
R¹² is the following:
1) normal alkyl having 1 to 12 carbon atoms, or branched alkyl having 3 to 14 carbon atoms;
2) -Z-Ar²
wherein Z is defined as the same as the above, and Ar² represents phenyl, α-naphthyl, β-naphthyl, or phenyl substituted by at least one chlorine; bromine, fluorine, iodine, trifluoromethyl, alkyl having 1 to 4 carbon atoms, nitro, cyano, methoxy, phenyl or phenoxy;
3) -CₜH₂ₜOR¹⁴
wherein CₜH₂ₜ is defined as the same as the above, and R¹⁴ represents normal alkyl having 1 to 6 carbon atoms, branched alkyl having 3 to 6 carbon atoms, phenyl, phenyl substituted by at least one chlorine, bromine, fluorine, iodine, trifluoromethyl, alkyl having 1 to 4 carbon atoms, nitro, cyano, methoxy, phenyl or phenoxy, cyclopentyl, cyclohexyl, or cyclopentyl or cyclohexyl substituted by 1 to 4 normal alkyl groups having 1 to 4 carbon atoms;
4) -Z-R³
wherein Z and R³ are defined as the same as the above;
5) -CₜH₂ₜ-CH=C(R¹⁵)R¹⁶
wherein CₜH₂ₜ is defined as the same as the above, and R¹⁵ and R¹⁶ each represent hydrogen, methyl, ethyl, propyl, or butyl; or
6) -CᵤH₂ᵤ-C≡C-R¹⁷
wherein u is an integer of 1 to 7, CᵤH₂ᵤ represents normal or branched alkyl, and R¹⁷ represents normal alkyl having 1 to 6 carbon atoms (when m = 3, branched ethylene C₂H₄ and methyl as R¹⁷ are excluded);
E is hydrogen or -OR¹⁸
wherein R¹⁸ represents acyl having 1 to 12 carbon atoms, aroyl having 7 to 15 carbon atoms, or R² (wherein R² is defined as the same as the above); and
the formula represents the d, l or dl form].

11. A cervical ripening method according to Claim 10, wherein A is -(CH₂)ₘ-, -CH=CH-CH₂-, -CH₂-CH=CH-, -CH₂-O-CH₂-, -CH=CH-, -O-CH₂-, or -C≡C- (m is 1 or 2).

12. A cervical ripening method according to Claim 10, wherein A is -(CH₂)₂-, -CH=CH-, or -O-CH₂-.
